# EUROPÄISCHE PATENTSCHRIFT

(11) **EP 0 576 936 B1**
(45) Veröffentlichungstag und Bekanntmachung des Hinweises auf die Patenterteilung: **25.11.1998**
(21) Anmeldenummer: 93109781.0
(22) Anmeldetag: 18.06.1993
(51) Int. Cl.: A61K 35/78

(54) **Hamamelis-Trockenextrakt, Verfahren zu seiner Herstellung und seine Verwendung als Arzneimittel**
Hamamelis dry extract, process for its preparation and its use as a medicine
Extrait sec d'Hamamelis, procédé de préparation et son utilisation comme médicament

(30) Priorität: 01.07.1992 DE 4221537
(43) Veröffentlichungstag der Anmeldung: 05.01.1994
(73) Patentinhaber: Dr. Willmar Schwabe GmbH & Co., D-76209 Karlsruhe (DE)
(72) Erfinder: Schwabe,Klaus-Peter,Dr.,, 7500 Karlsruhe 41 (DE)
(74) Vertreter: Bunke, Holger, Dr.rer.nat. Dipl.-Chem.

(56) Entgegenhaltungen:
- WO-A-90/13304
- FR-A- 2 578 744
- ARZNEIMITTEL FORSCHUNG. DRUG RESEARCH Bd. 28, Nr. 1, 1978, AULENDORF DE Seiten 1 - 7 G. MAY ET AL. 'ANTIVIRALE WIRKUNG W[SSRIGER PFLANZENEXTRAKTE IN GEWEBEKULTUREN'

## Beschreibung

Die Erfindung betrifft einen Trockenextrakt aus Pflanzenteilen von Hamamelis virginiana, ein Verfahren zu seiner Herstellung und diesen Extrakt enthaltende Arzneimittel.

Die Zaubernuß oder Hexenhasel Hamamelis virginiana ist seit langem als Arzneipflanze bekannt. Pharmakologisch wichtige Inhaltsstoffe sind die Gerbstoffe wie z. B. das Hamamelitannin, eine Digalloyl-α-hydroxymethyl-D-ribose, sowie freie Gallussäure und Flavonoide, die durch Extraktion der Rinde gewonnen werden, sowie die aus den Blättern durch Wasserdampfdestillation erhältlichen etherischen Öle. Ihre Wirksamkeit ist für leichte Hautverletzungen, lokale Entzündungen der Haut und Schleimhäute, Hämorrhoiden und Krampfaderbeschwerden anerkannt (BGA im Bundesanzeiger vom 21. August 1985, Seite 154). Daneben finden Hamamelis-Extrakte auch Anwendung in kosmetischen Präparaten.

Die antivirale Wirksamkeit einiger Gerbstoffe ist bereits bekannt. So beschreiben J. Courthout et al in Phytochemistry 30 (1991), S. 1129-1130, die Wirkung von Geraniin und Galloylgeraniin aus Spondias mombin auf Coxsackie B₂ und Herpes Simplex Typ 1 (HSV 1) Viren. K. Fukuchi et al (Antiviral Research 11 (1989), 285-298) sowie M. Takechi et al. (Phytochemistry 24 (1985), 2245-2250) berichten über die antivirale Wirksamkeit weiterer oligomerer, chemisch definierter Gerbstoffe, insbesondere gegenüber HSV1 und HSV2.

Schließlich wird in der PCT-Anmeldung FR90/00200 (WO 90/13304) eine Zusammensetzung pflanzlichen Ursprungs auf Proanthocyanidin-Basis beschrieben, bei der mindestens 50 Gew.-% der in ihr enthaltenen Moleküle ein relatives Molekulargewicht von über 2000 aufweisen, wobei von diesem Anteil etwa 40 Gew.-% der Moleküle mit Gerbfunktion aus Proanthocyanidinen bestehen. Diese Zusammensetzung ist im wesentlichen frei von etherischen Ölen und anderen lipophilen Substanzen und von Zuckern. Weitere Substanzen, die nicht in dieser Zusammensetzung enthalten sein sollen sind pflanzliche Proteine, Pyrogallol-Gerbstoffe, freie Gallussäure, Hydroxybenzoesäuren, Flavonoide, die nicht zu den Flavandiolen gehören, sowie Oligomere dieser Flavandiole. Als geeignete Pflanzen zur Extraktion dieser Gerbstoffe werden z. B. einige Koniferen-, Ingwer-, Quebracho- und Mimosenarten mit einem hohen Gehalt an kondensierten Gerbstoffen angesehen. Das in der obengenannten PCT-Anmeldung beschriebene Verfahren zur Isolierung dieser Zusammensetzung besteht darin, den zunächst gewonnenen alkoholischen Primärextrakt zur Trockene einzudampfen und anschließend mit mindestens drei Lösungsmitteln von unterschiedlicher Polarität in Gegenstromverteilung zu behandeln. Ein weiteres vorgeschlagenes Verfahren sieht vor, den alkoholischen Primärextrakt über eine Ultrafiltration zu reinigen, wobei die Ausschlußgrenze der verwendeten Membran für ein globuläres Protein in wäßriger Lösung bei 20.000 Dalton liegen soll. Die so erhaltenen Extrakte enthalten Proanthocyanidine mit einem Molekulargewicht von über 2000, und zeigen eine hohe Wirksamkeit gegenüber HSV 1 und einer Anzahl weiterer Viren bei gleichzeitig geringer Cytotoxizität.

Trotz der gut bekannten Anwendbarkeit bei den eingangs genannten Indikationen sind antivirale Wirkungen von wäßrigalkoholischen Hamamelis-Extrakten unbekannt. Ein auf die übliche Weise durch Extraktion von Hamamelis-Rinde (Hamamelis virginiana Cortex) mit Ethanol (76 Vol.-%) hergestellter Auszug besitzt nach Untersuchungen der Anmelderin keine spezifischen Hemmwirkungen gegenüber HSV-Viren. Es besteht daher ein Bedürfnis, neue Anwendungen für Hamamelis-Extrakte zu erschließen.

Aufgabe der Erfindung ist es daher, einen Hamamelis-Extrakt bereitzustellen, der zur Behandlung weiterer Indikationen geeignet ist, und unter anderem zur Herstellung von Arzneimitteln mit antiviralen Eigenschaften verwendet werden kann.

Diese Aufgabe wird erfindungsgemäß durch einen Hamamelis-Extrakt gemäß Patentanspruch 1 und durch das Verfahren zu seiner Herstellung gemäß den Patentansprüchen 2 bis 6 gelöst.

Es hat sich überraschenderweise gezeigt, daß der erfindungsgemäße Trockenextrakt antiviral, insbesondere anti-HSV1-wirksam ist, obwohl der bekannte wäßrig-alkoholische Primärauszug der Droge keine antivirale Wirksamkeit besitzt. Besonders überraschend ist dabei die geringe Cytotoxizität des erfindungsgemäßen Extrakts. Daneben zeigt der erfindungsgemäße Extrakt signifikante lokale antiphlogistische Wirkungen und Radikalfängereigenschaften in pharmakologischen Modellen.

Gegenstand der Erfindung sind auch Arzneimittel, insbesondere antiviral wirksame Arzneimittel, die den erfindungsgemäßen Trockenextrakt enthalten, gegebenenfalls zusammen mit üblichen, pharmakologisch verträglichen Hilfs- und Zusatzstoffen. Auch der Zusatz weiterer Wirkstoffe ist möglich, sofern diese Wirkstoffe mit dem erfindungsgemäßen Trockenextrakt kompatibel sind; Beispiele hierfür sind Wirkstoffe mit antiseptischen oder antiinflamatorischen Eigenschaften. Bei den pharmakologisch verträglichen Hilfs- und Zusatzstoffen kann es sich um übliche Salbengrundlagen, Tablettierhilfsstoffe, Farb-, Geschmacks-, Geruchsstoffe und dergleichen handeln.

Bei dem erfindungsgemäßen Verfahren zur Herstellung des Hamamelis-Trockenextrakts wird ein wäßrig-alkoholischer Auszug der Hamamelis-Droge, vorzugsweise der getrockneten Rinden, der nachfolgend "Primärextrakt" genannt wird, durch Ultrafiltration unter Verwendung einer Ultrafiltrationsmembran mit einer nominellen molekularen Ausschlußgrenze von 3000 Dalton fraktioniert, wonach das erhaltene Retentat, das die Inhaltsstoffe mit einem Molekulargewicht von wenigstens 3000 Dalton enthält, abgetrennt, von Lösungsmitteln befreit und getrocknet wird.

Das dabei erhaltene Filtrat, das Inhaltsstoffe mit einem Molekulargewicht von weniger als 3000 Dalton enthält, wird verworfen.

Das von der Ultrafiltrationsmembran zurückgehaltene Retentat wird anschließend in wäßrig-alkoholischer, vorzugsweise wäßrig-ethanolischer Lösung mit einem Ethanolgehalt von etwa 70 Vol.-%, aufgenommen, der Alkohol wird behutsam abgezogen, worauf schließlich getrocknet, vorzugsweise gefriergetrocknet wird. Das Retentat enthält die Inhaltsstoffe des Primärextrakts mit einem Molekulargewicht von wenigstens 3000 Dalton, darunter kondensierte Gerbstoffe vom Typ der Proanthocyanidine. Die Retentat-Ausbeute liegt, bezogen auf das Gewicht der als Ausgangsdroge verwendeten Hamamelis-Rinde, zwischen 4 und 12 %, vorzugsweise zwischen 5 und 8 %. Dem Retentat können gegebenenfalls übliche Hilfs- und Zusatzstoffe zugemischt werden.

Der erfindungsgemäß als Ausgangsmaterial eingesetzte Primärextrakt wird gewöhnlich durch Extraktion der Droge mit 50 bis 80 Vol.-%igem Alkohol, vorzugsweise Ethanol, hergestellt, anschließend getrocknet und für die Fraktionierung bzw. Ultrafiltration wieder in die Form einer wäßrig-alkoholischen Lösung gebracht. Hierzu wird der getrocknete Primärextrakt vorzugsweise in 25 %igem wäßrigem Ethanol gelöst, und zwar in solcher Menge, daß die Lösung einen Gehalt von 5 Gew.-% Primärextrakt aufweist.

Alternativ kann auf die Trocknung bzw. Isolierung des Primärextrakts verzichtet werden; in diesem Falle wird der Primärextrakt konzentriert, auf einen Ethanolgehalt von 25% und einen Trockenextraktgehalt von 5% eingestellt und dann die so erhaltene Lösung durch Ultrafiltration fraktioniert.

Der erfindungsgemäß erhaltene Trockenextrakt Zeigt neben den bereits genannten antiphlogistischen Eigenschaften eine hohe antivirale Wirksamkeit und gleichzeitig eine niedrige Cytotoxizität und kann vorteilhaft zur Herstellung von Arzneimitteln, beispielsweise zur Bekämpfung von Herpes Simplex-Viren, zur Therapie von Hautverletzungen, Entzündungen der Haut und der Schleimhäute und somit zur Wundheilung verwendet werden. Er läßt sich auf übliche Weise in eine Fett- oder Emulsionssalbe, ein Gel, einen Stift oder eine andere topische Darreichungsform einarbeiten.

Die Erfindung wird anhand des folgenden Ausführungsbeispiels weiter erläutert:

### Beispiel

### a) Herstellung eines Hamamelis-Rinden-Extrakts

Getrocknete, grob gepulverte Hamamelis-Rinde als Ausgangsdroge wird mit 68 bis 72 Vol.-%igem Ethanol bei etwa 58 - 60 °C extrahiert. Dieser Primärextrakt wird schonend am Vakuum getrocknet. Dabei wird ein erster Trockenextrakt mit einem Hamamelitannin-Gehalt von 20 % erhalten. Die Ausbeute an diesem ersten Trockenextrakt beträgt 19 bis 20 %, bezogen auf das Gewicht der Ausgangsdroge.

Dieser Trockenextrakt wird dann in 25 %igem wäßrigem Ethanol (V/V) gelöst, so daß eine 5 %ige Lösung erhalten wird. Diese Lösung wird dann über eine Ultrafiltrationsspiralpatrone mit einer unteren nominellen molekularen Trenngrenze von 3000 Dalton bei einem Druck von 1 bis 2 bar filtriert. Die Kalibrierung der Ultrafiltrationsmembran wird mit Hilfe von Cytochrom C ausgeführt, wobei die Rückhalterate an Cytochrom C größer als 90 % gewählt wird. Die zu filtrierende Lösung wird dabei jeweils auf etwa ein Drittel bis etwa auf die Hälfte des Ausgangsvolumens filtriert und anschließend mit 25 %igem Ethanol wieder auf das Ausgangsvolumen aufgefüllt. Auf diese Weise wird praktisch bis zur Farblosigkeit des Filtrats filtriert. Das Retentat wird mit 70 %igem Ethanol (V/V) aus der Spiralpatrone ausgewaschen, anschließend am Vakuum von Ethanol befreit und schließlich gefriergetrocknet.

Auf diese Weise werden durch Extraktion von 1750 g Hamamelis-Rinde etwa 350 g an getrocknetem Primärextrakt erhalten, woraus 102 g Retentat gewonnen werden, was einer Ausbeute von ca. 6 %, bezogen auf die Ausgangsdroge, entspricht.

### b) Antivirale Wirksamkeit der Hamamelis-Extrakte

Verschiedene Hamamelis-Extrakte bzw. Hamamelis-Inhaltsstoffe werden auf ihre Wirksamkeit (ID₅₀) gegen Herpes-Simplex Typ 1 Viren (HSV1) und auf ihre Cytotoxizität (CIC₅₀) untersucht und mit dem erfindungsgemäßen Extrakt verglichen. Zur weiteren Untermauerung der antiviralen Wirksamkeit wurden die genannten Zubereitungen auf Hemmung der α-Glucosidase untersucht (Literatur: R.R. Schmidt, H. Dietrich, Angew. Chem. 103 S. 1348-1349 (1991)). Die Ergebnisse sind in der nachfolgenden Tabelle 1 wiedergegeben.

Der darin angegebene Selektivitätsindex CIC₅₀/ID₅₀ (vgl. Huh and Hsiung, Antivir. Res. 11 (1989), 217-232) ermöglicht eine Aussage über das Maß der spezifischen antiviralen Wirkung eines Inhaltsstoffes oder -stoffgemisches und der mit diesen verbundenen Cytotoxizität. Selektivitätsindizes mit Werten über 1 zeigen, daß diese Stoffe eine deutlich spezifische antivirale Wirksamkeit besitzen.

**Tabelle 1**

| Extrakt | Antivirales Screeningmodell (HSV 1) | | | Plaque Reductionstest HSV 1 (IC 50 µg/ml) | α-Glucosidasehemmung (IC 50 µg/ml) |
|---|---|---|---|---|---|
| | Cytotoxizität (CIC 50) | Wirksamkeit (ID 50) | Selekt.Index (CIC 50/ID 50) | | |
| I. Erfindungsgemäßer Extrakt (Rotentat aus Beispiel a) | 74 µg/ml | 6,3 µg/ml | 11,7 | 6,7 | 0,53 |
| II. Vergleichsextrakt | | | | | |
| - Filtrat aus Beispiel a | 30 µg/ml | 16 µg/ml | 1,9 | 12,1 | 4,30 |
| - wäßrig ethanolischer Primärextrakt | 7,5 µg/ml | > 10 µg/ml | < 0,75 | - | 1,70 |
| - Hamamelitannin | 40,0 µg/ml | 26,0 µg/ml | 1,54 | - | 6,0 |

Die in der vorstehenden Tabelle dargestellten Ergebnisse zeigen, daß das erfindungsgemäß erhaltene Retentat überraschend gute Werte sowohl hinsichtlich der antiviralen Wirksamkeit als auch der Cytotoxizität aufweist. Der nach üblichen Methoden erhaltene Primärextrakt zeigt dagegen keine spezifische antivirale Wirkung. Das als Bestandteil von Hamamelis virginiana bekannte Hamamelitannin, das der Gruppe der hydrolysierbaren Gerbstoffe zuzurechnen ist, sowie das gemäß Beispiel a) erhaltene Filtrat zeigen ebenfalls keine befriedigende spezifische Wirksamkeit gegen HSV-1 und sind zur Herstellung von Arzneimitteln ungeeignet.

### c) Antiphlogistische Wirksamkeit

Der erfindungsgemäße Extrakt wurde im Crotonöl-Ohrödem-Modell bei der Maus auf antiphlogistische Wirksamkeit im Vergleich zu bekannten Antiphlogistika geprüft; dieses Modell dient als Nachweis der entzündungshemmenden Eigenschaften von Wirkstoffen. Ergebnisse:
- Erfindungsgemäßer Extrakt: ED 50 193 µg/Ohr
- Vergleich: Indometacin: ED 50 100 µg/Ohr

### d) Arzneimittelzubereitungen (in Gew.-%)

I)
   - 2,0 %: Hamamelisextrakt (Retentat nach Beispiel a)
   - 6,0 %: Polyethylenglycerinsorbitanisostearat
   - 13,50 %: Paraffin subl.
   - 4,00 %: Mikrowachs
   - 11,50 %: mittelkettige Triglyceride
   - 2,00 %: Glycerol (85%)
   - 5,00 %: Propylenglykol
   - 0,70 %: MgSO₄ x 7 H₂O
   - 55,30 %: Aqua purificata (DAB 10)
   werden miteinander vermischt und in bekannter Weise in eine topische Darreichungsform eingearbeitet.
II) Salbenzubereitung, bestehend aus:
   - 2,0 %: Hamamelisextrakt (Retentat nach Beispiel a)
   - 6,0 %: Polyethylenglykolsorbitanisostearat
   - 13,5 %: Paraffin
   - 4,0 %: Mikrowachs
   - 11,5 %: mittelkettige Triglyceride
   - 5,0 %: Propylenglykol
   - 1,0 %: Benzylalkohol
   - 2,0 %: Lecithin
   - 0,7 %: MgSO₄·7 H₂O
   - 54,3 %: Aqua purificata (DAB 10)

## Patentansprüche

1. Trockenextrakt aus Pflanzenteilen von Hamamelis virginiana, dadurch gekennzeichnet, daß er, gegebenenfalls neben üblichen Hilfs- und Zusatzstoffen, ausschließlich solche Inhaltsstoffe eines wäßrig-alkoholischen Auszugs der Hamamelis-Droge enthält, die durch eine Ultrafiltrationsmembran mit einer nominellen molekularen Trenngrenze von 3000 Dalton zurückgehalten werden.

2. Verfahren zur Herstellung des Trockenextrakts gemäß Anspruch 1, dadurch gekennzeichnet, daß ein wäßrig-alkoholischer Auszug der Hamamelis-Droge (Primärextrakt) durch Ultrafiltration unter Verwendung einer Ultrafiltrationsmembran mit einer nominellen molekularen Ausschlußgrenze von 3000 Dalton fraktioniert wird, daß das erhaltene Retentat, das die Inhaltsstoffe mit einem Molekulargewicht von wenigstens 3000 Dalton enthält, abgetrennt, von Lösungsmitteln befreit und getrocknet wird.

3. Verfahren nach Anspruch 2, dadurch gekennzeichnet, daß das Retentat in einer wäßrig-ethanolischen Lösung mit einem Ethanolgehalt nolgehalt von etwa 70 Vol.-% aufgenommen und anschließend getrocknet wird.

4. Verfahren nach Anspruch 3, dadurch gekennzeichnet, daß die wäßrig-ethanolische Lösung anschließend gefriergetrocknet wird.

5. Verfahren nach einem der Ansprüche 2 bis 4, dadurch gekennzeichnet, daß der Primärextrakt getrocknet und in 25 %igem (V/V) wäßrigem Ethanol gelöst wird, wonach diese Lösung fraktioniert wird.

6. Verfahren nach einem der Ansprüche 2 bis 4, dadurch gekennzeichnet, daß der Primärextrakt konzentriert, auf 25% Ethanolgehalt und 5% Trockenextraktgehalt eingestellt wird, und daß die so erhaltene Lösung fraktioniert wird.

7. Arzneimittel, enthaltend einen Extrakt gemäß Anspruch 1, ggf. zusammen mit üblichen, pharmakologisch verträglichen Hilfs- und Zusatzstoffen.

8. Verwendung eines Extrakts gemäß Anspruch 1 zur Herstellung von Arzneimitteln zur Bekämpfung von Herpes Simplex-Viren und zur Wundheilung.

## Claims

1. A dry extract from plant parts of hamamelis virginiana characterized in that said extract exclusively contains, optionally apart from conventional adjuvants and additives, those components of an aqueous-alcoholic extract of the hamamelis drug which are retained by an ultrafiltration membrane having a nominal molecular separation limit of 3000 Daltons.

2. A process for the manufacture of the dry extract according to claim 1 characterized in that an aqueous-alcoholic extract of the hamamelis drug (primary extract) is fractionated by means of ultrafiltration using an ultrafiltration membrane having a nominal molecular exclusion limit of 3000 Daltons, and that the obtained retentate containing the components having a molecular weight of at least 3000 Daltons is separated, liberated from solvents, and dried.

3. The process of claim 2 characterized in that said retentate is taken up into an aqueous-ethanolic solution having a content of approximately 70 vol.-% ethanol and, subsequently, is dried.

4. The process of claim 3 characterized in that said aqueous-ethanolic solution subsequently is lyophilized.

5. The process of any one of claims 2 to 4 characterized in that said primary extract is dried and dissolved in aqueous 25% (v/v)-ethanol, whereafter this solution is fractionated.

6. The process of any one of claims 2 to 4 characterized in that said primary extract is concentrated, adjusted to contents of 25% ethanol and 5% dry extract, and in that the solution thus obtained is fractionated.

7. A pharmaceutical composition containing an extract according to claim 1, optionally together with conventional, pharmacologically acceptable adjuvants and additives.

8. Use of an extract according to claim 1 for the manufacture of pharmaceuticals for controlling herpes simplex-viruses and for wound-healing.

## Revendications

1. Extrait sec issu de parties végétales de l'Hamamelis virginiana, caractérisé par le fait qu'il contient le cas échéant, outre les substances auxiliaires et additionnelles usuelles, exclusivement des constituants d'un extrait alcoolo-aqueux de la drogue Hamamelis, ayant été retenu au moyen d'une membrane à ultrafiltration d'une limite de séparation moléculaire nominale de 3000 Dalton.

2. Procédé de préparation de l'extrait sec selon la revendication 1, caractérisé par le fait qu'on fractionne un extrait alcoolo-aqueux de la drogue Hamamelis (extrait primaire) par ultrafiltration avec utilisation d'une membrane d'ultrafiltration d'une limite d'exclusion moléculaire nominale de 3000 Dalton, et que la matière retenue obtenue, qui contient les constituants ayant un poids moléculaire d'au moins 3000 Dalton, est séparée, débarrassée des solvants et séchée.

3. Procédé selon la revendication 2, caractérisé par le fait que le rétentat est capté dans une solution éthanolo-aqueuse ayant une teneur en éthanol d'environ 70 % en volume, puis est séché.

4. Procédé selon la revendication 3, caractérisé par le fait que la solution éthanolo-aqueuse est ensuite séchée par congélation.

5. Procédé selon l'une des revendications 2 à 4, caractérisé par le fait que l'extrait primaire est séché et est dissous dans une solution aqueuse d'éthanol à 25 % (V/V), puis cette solution est fractionnée.

6. Procédé selon l'une des revendications 2 à 4, caractérisé par le fait que l'extrait primaire est concentré, réglé à une teneur en éthanol de 25 % et à une teneur en extrait sec de 5 %, et que la solution ainsi obtenue est fractionnée.

7. Médicament, contenant un extrait selon la revendication 1, le cas échéant conjointement avec des substances auxiliaires et additionnelles usuelles, compatibles pharmacologiquement.

8. Utilisation d'un extrait selon la revendication 1, pour la préparation de médicaments pour la lutte contre les virus simplex de l'herpès et pour la guérison des blessures.
